(19) Europäisches Patentamt
European Patent Office
Office européen des brevets

(11) **EP 3 831 274 A1**

(12) **EUROPEAN PATENT APPLICATION**
published in accordance with Art. 153(4) EPC

(43) Date of publication:
**09.06.2021 Bulletin 2021/23**

(21) Application number: **19797969.3**

(22) Date of filing: **26.06.2019**

(51) Int Cl.:
**A61B 3/10** (2006.01)

(86) International application number:
**PCT/JP2019/025379**

(87) International publication number:
**WO 2020/026652 (06.02.2020 Gazette 2020/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(30) Priority: **31.07.2018 JP 2018143802**

(71) Applicant: **Rexxam Co., Ltd.
Osaka-shi, Osaka 541-0054 (JP)**

(72) Inventors:
• **IWAMOTO, Masakatsu
Takamatsu-shi, Kagawa 761-1494 (JP)**

• **YAMAMOTO, Daichi
Takamatsu-shi, Kagawa 761-1494 (JP)**
• **KAWAI, Jun
Takamatsu-shi, Kagawa 761-1494 (JP)**
• **TAKATA, Tomohito
Takamatsu-shi, Kagawa 761-1494 (JP)**
• **ISEKI, Yuji
Takamatsu-shi, Kagawa 761-1494 (JP)**

(74) Representative: **Betten & Resch
Patent- und Rechtsanwälte PartGmbB
Maximiliansplatz 14
80333 München (DE)**

(54) **OPHTHALMIC DEVICE**

(57) In order to reduce the influence of a change between base amounts of light of an iris region and a pupil region in measurement regarding the state of a lacrimal fluid layer on a cornea surface of an examination target eye, an ophthalmic apparatus 1 for performing measurement regarding the state of a lacrimal fluid layer on a cornea surface of an examination target eye 2 includes: a light-projecting unit 13 that projects a ring pattern onto a cornea surface; an image capturing unit 14 that captures a reflected image of the ring pattern reflected off the cornea surface; a calculating unit 15 that calculates a bluntness level indicating a bluntness level at a maximum portion of luminance values in a direction that is orthogonal to the ring pattern of the reflected image captured by the image capturing unit 14; and an output unit 16 that performs output regarding the bluntness level calculated by the calculating unit 15. If a bluntness level is calculated in this manner according to the shape of a peak of luminance values, it is possible to reduce the influence of a change between base amounts of light of an iris region and a pupil region.

FIG.1

**Description**

Technical Field

The present invention relates to an ophthalmic apparatus for performing measurement regarding the state of a lacrimal fluid layer on a cornea surface of an examination target eye.

Background Art

Conventionally, there are known ophthalmic apparatuses for performing measurement regarding the state of a lacrimal fluid layer formed on a cornea surface of an examination target eye (see Patent Document 1). The ophthalmic apparatus described in Patent Document 1 performs evaluation regarding the state of a lacrimal fluid layer, such as evaluation of dry eye syndrome or the like, using a density value distribution (luminance value distribution) regarding a reflected image of predetermined pattern light projected onto a cornea surface of an examination target eye.

Citation List

Patent Document

Patent Document 1: JP 2006-204773A

Summary of Invention

Technical Problem

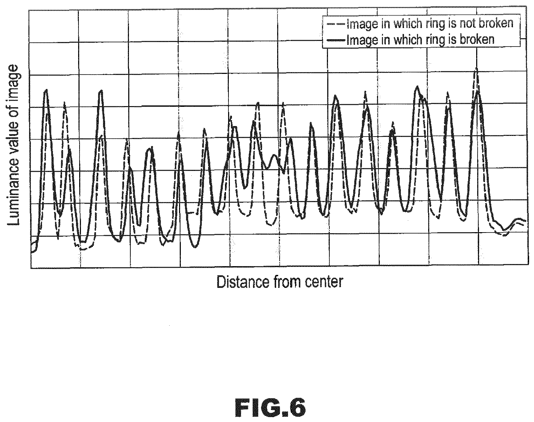

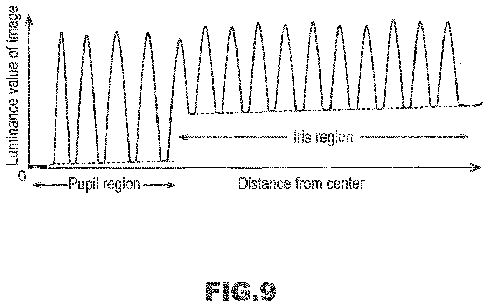

An iris region and a pupil region of an examination target eye have different base amounts of light in a reflected image of pattern light. FIG. 9 is a schematic graph illustrating a difference between base amounts of light of an iris region and a pupil region. The measuring method in FIG. 9 is similar to that in FIG. 6, which will be described later. In FIG. 9, the base amounts of light are indicated by the broken lines. As can be seen from FIG. 9, the base amount of light of an iris region is higher than the base amount of light of a pupil region. Accordingly, even when the state of a lacrimal fluid layer does not change between an iris region and a pupil region, measurement using a density value distribution including background reflected light from the iris results in, for example, the contrast (peaks and valleys of luminance values) in the iris region being different from the contrast in the pupil region, and thus it is not possible to correctly measure the state of a lacrimal fluid layer.

Furthermore, when observing the state of a lacrimal fluid layer, the measurement may be performed continuously for a constant period of time (e.g., 10 seconds, 15 seconds, etc.). If pattern light is projected onto an examination target eye for a constant period of time in this manner, the pupil size changes due to the influence of the pattern light. As a result, there may be a measurement point that is located in a pupil region at the measurement start time but comes to be located in an iris region during the measurement period. At the measurement point whose location changes from the pupil region to the iris region during the measurement period in this manner, the base amount of light changes even if the lacrimal fluid layer does not change, and thus the density value distribution regarding a reflected image containing the measurement point also changes. As a result, evaluation may be inaccurate.

Generally, there is a problem in that, when performing measurement regarding the state of a lacrimal fluid layer using a luminance value distribution regarding a reflected image of pattern light projected onto a cornea surface of an examination target eye, accurate measurement cannot be performed due to a difference between base amounts of light of an iris region and a pupil region.

The present invention was made in order to solve the above-described problem, and it is an object thereof to provide an ophthalmic apparatus in which, when performing measurement regarding the state of a lacrimal fluid layer on a cornea surface of an examination target eye, it is possible to reduce the influence of a change between base amounts of light of an iris region and a pupil region.

Solution to Problem

In order to achieve the above-described object, the present invention is directed to an ophthalmic apparatus for performing measurement regarding the state of a lacrimal fluid layer on a cornea surface of an examination target eye, including: a light-projecting unit that projects a predetermined pattern onto a cornea surface; an image capturing unit that captures a reflected image of the pattern reflected off the cornea surface; a calculating unit that calculates bluntness information indicating a bluntness level at a maximum portion of luminance values in the reflected image captured by the image capturing unit; and an output unit that performs output regarding the bluntness information calculated by the calculating unit.

With this configuration, it is possible to perform measurement regarding the state of a lacrimal fluid layer, using bluntness information of a maximum portion of luminance values in a reflected image. Accordingly, it is possible to perform proper measurement regardless of a change between base amounts of light of an iris region and a pupil region.

Furthermore, the ophthalmic apparatus according to the present invention may be such that the image capturing unit repeatedly captures reflected images of the pattern, the calculating unit calculates bluntness information for each of the reflected images that were repeatedly captured, and the output unit outputs information indicating a time-series change in the bluntness information.

With this configuration, it is possible to see a time-series change in the lacrimal fluid layer. In this case, it is possible to perform proper measurement also in a region in which the pupil size changes, that is, a region that is a pupil region when measurement is started but that changes into an iris region during the measurement period.

Furthermore, the ophthalmic apparatus according to the present invention may be such that the predetermined pattern is a ring pattern, the calculating unit calculates bluntness information at an intersection between the ring pattern and each straight line radially extending at every predetermined angle from a center position specified in a reflected image of the ring pattern, and the output unit performs output regarding the multiple pieces of bluntness information calculated by the calculating unit.

With this configuration, for example, it is possible to calculate bluntness information of the entire cornea surface of an examination target eye, by using a pattern of multiple rings.

Advantageous Effects of Invention

With the ophthalmic apparatus according to the present invention, measurement regarding the state of a lacrimal fluid layer is performed, by calculating bluntness information indicating a bluntness level at a maximum portion of luminance values, instead of using luminance values themselves in a reflected image of a pattern. Accordingly, it is possible to reduce the influence of a change between base amounts of light of a pupil region and an iris region.

Brief Description of Drawings

FIG. 1 is a schematic diagram showing the configuration of an ophthalmic apparatus according to an embodiment of the present invention.
FIG. 2 is a flowchart illustrating an operation of the ophthalmic apparatus in this embodiment.
FIG. 3 is a diagram illustrating measurement of luminance values in this embodiment.
FIG. 4 shows examples of an examination target eye and the like onto which a pattern is projected in this embodiment.
FIG. 5 shows examples of an examination target eye and the like onto which a pattern is projected in this embodiment.
FIG. 6 is a graph showing an example of a change in luminance values in the radial direction of an examination target eye in this embodiment.
FIG. 7 is a diagram showing an example of the luminance in a direction that is orthogonal to a ring pattern in this embodiment.
FIG. 8 is a diagram showing an example of a time-series change in the total of bluntness levels in this embodiment.
FIG. 9 is a schematic graph illustrating a difference between base amounts of light of a pupil region and an iris region.

Description of Embodiment

Hereinafter, an ophthalmic apparatus according to the present invention will be described using an embodiment. It should be noted that constituent elements and steps denoted by the same reference numerals in the following embodiments are the same or corresponding constituent elements and steps, and thus a description thereof may not be repeated. The ophthalmic apparatus according to this embodiment calculates bluntness information of a maximum portion of luminance values of a reflected image of a pattern projected onto a cornea surface.

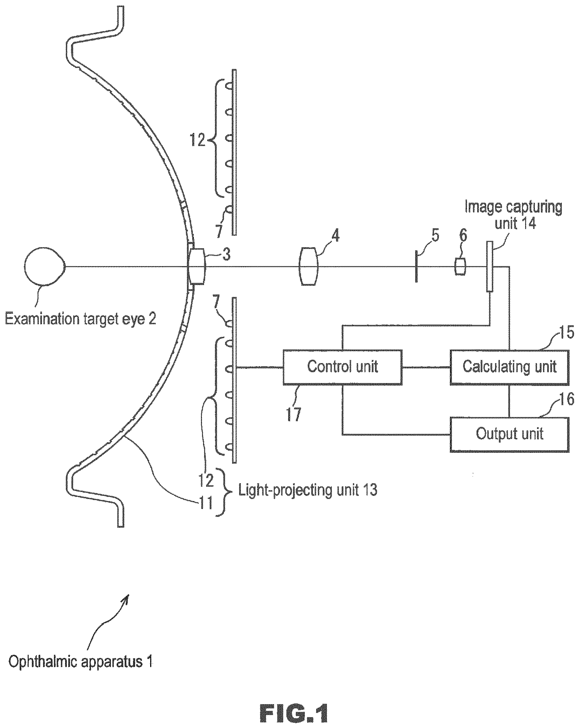

FIG. 1 is a schematic diagram showing the configuration of an ophthalmic apparatus 1 according to this embodiment. The ophthalmic apparatus 1 according to this embodiment performs measurement regarding the state of a lacrimal fluid layer on a cornea surface of an examination target eye 2, and includes the configuration of an optical system having an ocular lens 3, a field lens 4, a diaphragm (pinhole) 5, and an imaging lens 6, an illumination light source 7, a light-projecting unit 13, an image capturing unit 14, a calculating unit 15, an output unit 16, and a control unit 17. The ophthalmic apparatus 1 may perform, for example, only measurement regarding the state of a lacrimal fluid layer, or may have the function of a keratometer or the like.

The illumination light source 7 is a light source for illuminating the examination target eye 2, and illuminates the examination target eye 2 in order to allow an examiner to see the state of an eyelid of an examinee or the like. The illumination light emitted from the illumination light source 7 may be, for example, far infrared light.

The light-projecting unit 13 projects a predetermined pattern onto a cornea surface of the examination target eye 2. The predetermined pattern may be, for example, a linear pattern or a dotted pattern, or may be a combination thereof. The linear pattern may be, for example, a pattern having one line, or may be a pattern having multiple lines. The line may be for example, a curved line, or may be a straight line. A pattern having a curved line may be, for example, a ring pattern having one ring, or may be a pattern of multiple rings having multiple concentric rings. The dotted pattern may be, for example, a pattern having one dot (point), or may be a pattern having multiple points. A pattern having multiple points may be, for example, a pattern having multiple points that are arranged regularly, or may be a pattern having multiple points that are arranged randomly. In the case of the former, the pattern having multiple points may be, for example, a set of multiple points that are arranged at lattice points on a square lattice, a rectangular lattice, a triangular lattice, or the like. It is preferable that, in a predetermined pattern, multiple lines have the same width, and multiple points have the same diameter. In this embodiment, a case will mainly be described in which the light-projecting unit 13 has a placido dome 11 and a measurement light source 12, and the predetermined pattern is a pattern having multiple concentric rings, that is, a pattern of multiple rings (a placido ring) formed by the placido dome 11. The placido dome 11 is a dome-like optical mask having openings in the shape of multiple concentric rings, through which measurement light emitted from the measurement light source 12 forms a ring pattern, which is a pattern having multiple concentric rings, on the anterior eye part of the examination target eye 2. There is no limitation on the wavelength of the measurement light emitted from the measurement light source 12. The measurement light may be, for example, visible light, or may be near infrared light or the like. If the measurement light is visible light, the wavelength may be, for example, 650 nm, 750 nm, or the like. There is no limitation on the method for projecting a ring pattern onto the examination target eye 2. The process of projecting a ring pattern onto the examination target eye 2 is already known, and a detailed description thereof has been omitted.

Each of the illumination light source 7 and the measurement light source 12 may be arranged in a ring-like form about the optical axis of the optical system.

The ring pattern projected onto the cornea surface of the examination target eye 2 is reflected off the cornea surface. The reflected pattern is transmitted through the ocular lens 3, the field lens 4, the diaphragm 5, and the imaging lens 6, and forms an image. The image capturing unit 14 captures a reflected image of the pattern. The image capturing unit 14 may be, for example, a CCD image sensor, a CMOS image sensor, or the like. The image capturing unit 14 may repeatedly capture reflected images of a pattern. The images may be repeatedly captured at predetermined time intervals.

The calculating unit 15 calculates bluntness information indicating a bluntness level at a maximum portion of luminance values in the reflected image captured by the image capturing unit 14. If the predetermined pattern has a line, the calculating unit 15 may calculate bluntness information of a maximum portion of luminance values, in a direction that is orthogonal to the line in the captured reflected image. If the predetermined pattern has a point, the calculating unit 15 may calculate bluntness information of a maximum portion of luminance values, in any direction that passes through the point in the captured reflected image. It is preferable that bluntness information regarding a point is bluntness information on a straight line that passes through the center of the point in the reflected image. If a reflected image of a ring pattern is captured, the line in the reflected image is in the shape of a ring. Accordingly, if a reflected image of a ring pattern is captured, the calculating unit 15 specifies a center position in the reflected image of the ring pattern. The center position may be specified, for example, by specifying the center of the ring with the smallest diameter contained in the pattern of multiple rings. The calculating unit 15 calculates bluntness information at the intersection between the ring pattern and each straight line radially extending at every predetermined angle from the specified center position.

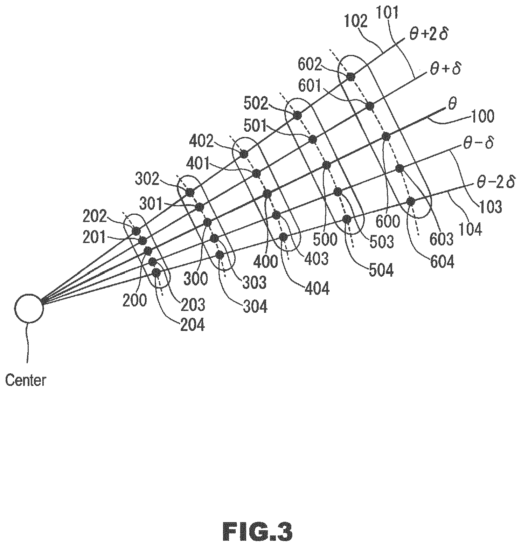

Hereinafter, a method in which the calculating unit 15 acquires data in the direction of a straight line radially extending from the specified center position will be described. FIG. 3 is a diagram illustrating acquisition of the data. In FIG. 3, when sampling a luminance value at a point on a straight line radially extending at an angle $\theta$ from the specified center position, the calculating unit 15 may use luminance values of nearby points at the same distance from the center position. The luminance values of nearby points may be, for example, luminance values on straight lines radially extending from the center position at every $\delta$ from an angle $\theta-n\times\delta$ to an angle $\theta+n\times\delta$. Note that n is an integer of 1 or more, and $\delta$ is a positive real number. In FIG. 3, n = 2. If the calculating unit 15 acquires data in the direction of a straight line radially extending from the specified center position at angle intervals $\Delta\theta$, it is preferable that $n\times\delta < \Delta\theta/2$. There is no particular limitation on $\Delta\theta$, but it may be, for example, 5 degrees, 10 degrees, 15 degrees, or the like. If $\Delta\theta$ is 10 degrees, luminance values on 36 straight lines radially extending from the center position are sampled. When acquiring a luminance value at the angle $\theta$ in this manner, the calculating unit 15 may acquire, as the luminance value at the angle $\theta$, a representative value of 2n+1 luminance values consisting of those at the angles $\theta-n\times\delta$, $\theta-(n-1)\times\delta$, ..., $\theta$, ..., $\theta+(n-1)\times\delta$, and $\theta+n\times\delta$. The representative value may be, for example, a mean, a median, a maximum, or the like. Specifically, in FIG. 3, when acquiring a luminance value at a point on a straight line 100 at the angle $\theta$, the calculating unit 15 may use a luminance value on a straight line 101 at an angle $\theta+\delta$, a luminance value on a straight line 102 at an angle $\theta+2\delta$, a

luminance value on a straight line 103 at an angle θ-δ, and a luminance value on a straight line 104 at an angle θ-2δ. That is to say, when acquiring a luminance value at a position 200, the calculating unit 15 may acquire a representative value of luminance values at positions 200 to 204. In a similar manner, the calculating unit 15 may acquire, as luminance values on the straight line 100 corresponding to the angle θ, a representative value of luminance values at positions 300 to 304, a representative value of luminance values at positions 400 to 404, a representative value of luminance values at positions 500 to 504, a representative value of luminance values at positions 600 to 604, and the like sequentially from the center side. If the data is acquired in this manner, for example, data of a reflected image even at an angle at which the reflected image of a ring pattern is incomplete can be acquired from data at nearby angles, and the position of the intersection between the radially extending straight line and the ring can be specified. In the description above, a case was described in which the calculating unit 15 acquires, as a luminance value on a straight line at a given angle θ, a representative value of multiple luminance values including luminance values of nearby points, but there is no limitation to this. For example, in FIG. 3, the calculating unit 15 may sample, as data of luminance values on the straight line 100, a luminance value at the position 200, a luminance value at the position 300, a luminance value at the position 400, and the like sequentially from the center side. The calculating unit 15 may acquire, as luminance values on radially extending straight lines, luminance values on straight lines radially extending at every Δθ from the specified center position.

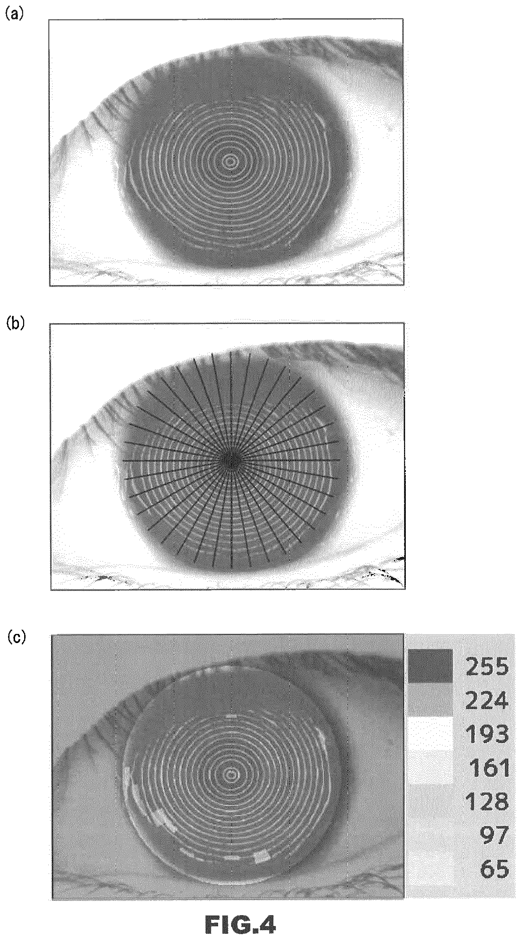

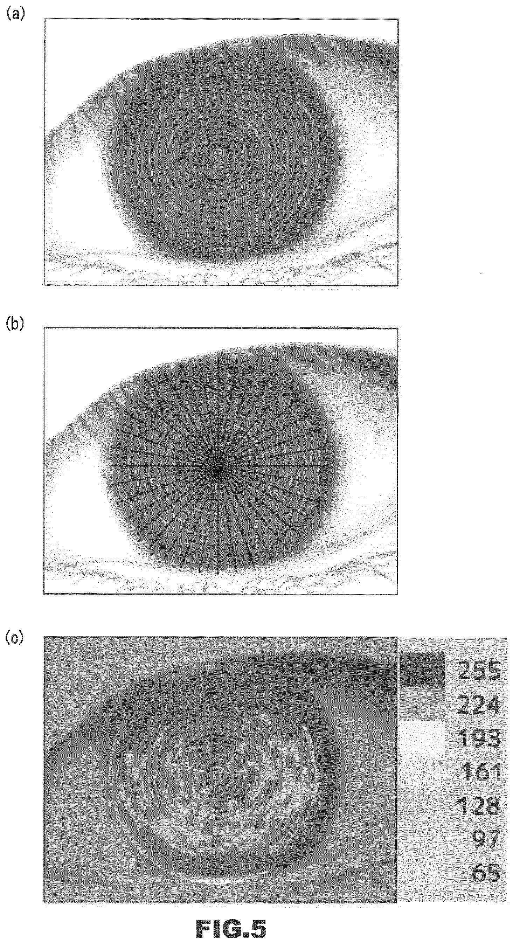

FIG. 4 shows examples of captured images of the examination target eye 2 in which a lacrimal fluid layer on a cornea surface is not broken, and FIG. 5 shows examples of captured images of the examination target eye 2 in which a lacrimal fluid layer on a cornea surface is broken. More specifically, FIGS. 4(a) and 5(a) are captured images of a reflected image of a ring pattern reflected off the cornea surface, FIGS. 4(b) and 5(b) are viewed in which straight lines radially extending at every predetermined angle from the center position are superimposed on the captured images, and FIGS. 4(c) and 5(c) are captured images on which colors each corresponding to the calculated bluntness information are superimposed. In the case of a pattern of multiple rings shown in FIGS. 4(a) and 5(a), when image capturing is started, for example, 5 or 6 rings from the inner side are located inside the pupil of the examination target eye 2.

As is clear from a comparison between FIGS. 4(a) and 5(a), if the lacrimal fluid layer is not broken, the rings of the pattern of multiple rings in the reflected image are not broken, whereas, if the lacrimal fluid layer is broken, the rings of the pattern of multiple rings in the reflected image are broken. FIG. 6 shows a relationship between the distance from the center of reflected images of a ring pattern and the luminance values, in a predetermined angular direction. The luminance values shown in FIG. 6 were obtained, for example, through sampling in the above-described manner from a captured image of an examination target eye. In FIG. 6, the change in the luminance values regarding an image in which the rings are not broken, that is, an image of an examination target eye in which the lacrimal fluid layer is not broken is indicated by the broken lines, and the change in the luminance values regarding an image in which the rings are broken, that is, an image of an examination target eye in which the lacrimal fluid layer is broken is indicated by the solid line. In FIG. 6, the luminance values have their peaks at positions of the rings in the reflected images. As is clear from FIG. 6, the shape of the peaks of the luminance values regarding the examination target eye in which the lacrimal fluid layer is broken is blunter than the shape of the peaks of the luminance values regarding the examination target eye in which the lacrimal fluid layer is not broken. In other words, the shape of the peaks of the luminance values regarding the examination target eye in which the lacrimal fluid layer is not broken is sharper than the shape of the peaks of the luminance values regarding the examination target eye in which the lacrimal fluid layer is broken. Accordingly, it is possible to see the level to which the lacrimal fluid layer is broken, by calculating bluntness information indicating the bluntness level of the shape of the peaks of the luminance values, and thus the calculating unit 15 calculates the bluntness information. There is no limitation on the bluntness information calculated by the calculating unit 15, as long as it is eventually possible to see the bluntness level of a maximum portion (a peak) of luminance values of a reflected image of a pattern. The bluntness information may be, for example, a bluntness level indicated by a value that increases in accordance with an increase in the bluntness level, or may be a sharpness level indicated by a value that decreases in accordance with an increase in the bluntness level. In this embodiment, a case will mainly be described in which the bluntness information is a bluntness level. Accordingly, in the description below, a description may be given assuming that the bluntness information is a bluntness level.

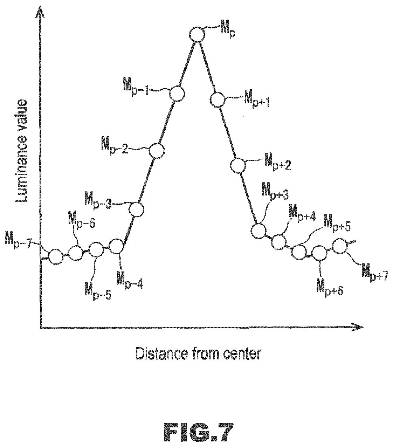

Next, a method in which the calculating unit 15 calculates a bluntness level will be described. FIG. 7 is a graph showing a relationship between the luminance values regarding one peak and the distance from the center, as acquired in a direction along a straight line at the angle θ. In FIG. 7, each circle mark indicates a relationship between the luminance value at a sampling point and the distance from the center. It is assumed that a sampling point $M_p$ corresponds to a maximum value, wherein the subscript increases or decreases in a direction away from the sampling point $M_p$. The calculating unit 15 may calculate a bluntness level B regarding a peak using the following formula.

$$B = b - a \sum_{m=-k}^{k} |I_p - I_{p+m}|$$

<div align="right">Numerical Formula 1</div>

In the formula, a luminance value at a sampling point $M_{p+d}$ is $I_{p+d}$. d is any integer. Accordingly, for example, $I_p$ is a luminance value that is a maximum value. Each of a and b is a constant that is a positive real number, and k is an integer of 1 or more. It is possible to change the influence (sensitivity) of the shape of a peak of luminance values on the bluntness level, by changing the value of a. The total in the formula above increases in accordance with an increase in the sharpness of a peak of luminance values, and thus, it is possible to increase the sensitivity by increasing the value of a, and it is possible to decrease the sensitivity by decreasing the value of a. It is preferable that the constant b is determined as appropriate, for example, such that the bluntness level B has a positive value. Furthermore, it is also preferable that k is set to a value at which the shape of a peak of luminance values is properly included, according to the intervals of sampling points of luminance values along the straight line at the angle θ. For example, in the case of FIG. 7, k may be set to 2.

The shape of a peak of luminance values in a direction that is orthogonal to a ring does not significantly change even when the baseline of luminance values changes between an iris region and a pupil region. Accordingly, it is possible to reduce the influence of a change between baselines of luminance values of an iris region and a pupil region, by calculating bluntness information using the shape of a peak of the luminance values, and thus it is possible to realize proper measurement regarding the state of a lacrimal fluid layer on a cornea surface of an examination target eye.

The method for calculating the bluntness level B is merely an example, and it is also possible to calculate a bluntness level using other methods. For example, it is also possible to calculate a bluntness level using a slope of multiple luminance values from a peak to the center side and a slope of multiple luminance values from the peak to the outer side.

Furthermore, the bluntness level B is a value regarding one peak of luminance values, but, as shown in FIG. 6, there are multiple peaks on a straight line at a given angle radially extending from the center position of a reflected image of a pattern of multiple rings, and there is such a straight line for every angle Δθ. Accordingly, the calculating unit 15 may calculate bluntness information for each peak of luminance values, on each straight line radially extending at every predetermined angle from the center position of a reflected image of a pattern of multiple rings. In this manner, it is possible to calculate bluntness information at every measurement point over the entire cornea of an examination target eye. The position of each measurement point is the position of the intersection between each straight line radially extending at equal intervals from the center position of the reflected image, and rings of the pattern of multiple rings. The calculating unit 15 may calculate bluntness information, for example, in a predetermined range or region in the cornea of an examination target eye. It is also possible that the calculating unit 15 acquires the total value, a representative value, or the like of multiple pieces of bluntness information of an image captured at a given point in time. The representative value may be, for example, a mean, a median, a maximum, or the like. If reflected images of a pattern are repeatedly captured, the calculating unit 15 may calculate multiple pieces of bluntness information at measurement points on the cornea, in each reflected image of the repeatedly captured images, and may acquire a total value, a representative value, or the like based on the multiple pieces of bluntness information.

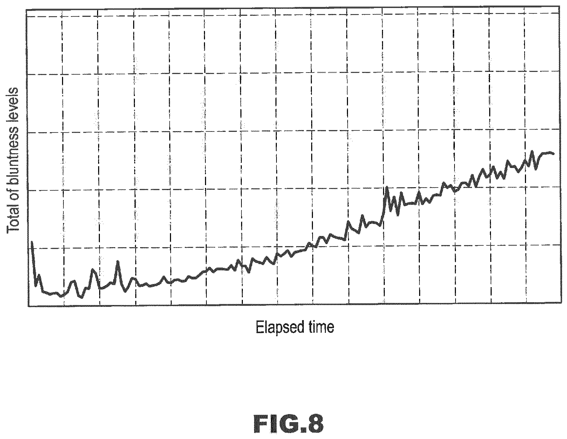

The output unit 16 performs output regarding the bluntness information calculated by the calculating unit 15. The output may be, for example, output of bluntness information itself, or may be output of a judgment result, an evaluation result, or the like regarding the bluntness information. If output regarding bluntness information is performed, a measurement result regarding a lacrimal fluid layer on a cornea surface of an examination target eye is output. If multiple pieces of bluntness information are calculated, the output unit 16 performs output regarding the multiple pieces of bluntness information. The output unit 16 may output, for example, multiple pieces of bluntness information themselves, or may output a total value or a representative value of the multiple pieces of bluntness information. The total value or the representative value may be calculated by the calculating unit 15. For example, the output unit 16 may display an image in which colors each corresponding to a range of values of the bluntness information are superimposed on a captured image of an examination target eye, at a position of the measurement point corresponding to the bluntness information, as shown in FIGS. 4(c) and 5(c). With this configuration, it is easy to see where the lacrimal fluid layer is broken. If the image capturing unit 14 repeatedly captures reflected images of a pattern and the calculating unit 15 calculates bluntness information for each captured image, the output unit 16 may output information indicating a time-series change in the bluntness information. The information indicating a time-series change in the bluntness information may be, for example, a graph showing a time-series change in the bluntness information, or may be information indicating the bluntness information at each image capturing point in time. Specifically, the output unit 16 may output a graph showing the total of bluntness levels (the total value) according to the elapsed time as shown in FIG. 8. If the time-series change in the bluntness information is output, a time-series change regarding the state of a lacrimal fluid layer of an examination target eye can be seen.

The output may be, for example, display on a display device (e.g., a liquid crystal display, an organic EL display, etc.), transmission via a communication line to a predetermined device, printing by a printer, sound output by a speaker, accumulation in a storage medium, or delivery to another constituent element. The output unit 16 may or may not include a device that performs output (e.g., a display device, a printer, etc.). The output unit 16 may be realized by hardware, or may be realized by software such as a driver that drives these devices.

The control unit 17 performs control of processing timings and the like regarding on/off of the measurement light source 12, image capturing by the image capturing unit 14, calculation of bluntness information by the calculating unit 15, output by the output unit 16, and the like.

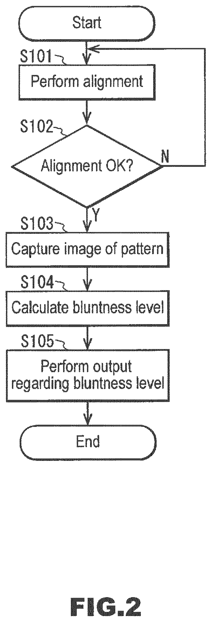

Next, an operation of the ophthalmic apparatus 1 will be described with reference to the flowchart in FIG. 2. The illumination light source 7 may be on during the measurement period regarding bluntness information. In this flowchart, it is assumed that the calculating unit 15 calculates multiple pieces of bluntness information of one captured image.

(Step S101) Alignment that aligns the examination target eye 2 and the optical system of the ophthalmic apparatus 1 in a proper positional relationship is performed. This alignment processing may be manually performed, or automatically performed.

(Step S102) The control unit 17 judges whether or not the alignment has been completed. If the alignment has been completed, the procedure advances to step S103, and, if not, the procedure returns to step S101. The judgment may be performed, for example, using a captured image acquired by the image capturing unit 14.

(Step S103) The control unit 17 turns on the measurement light source 12. As a result, a ring pattern is projected onto the cornea surface of the examination target eye 2. The control unit 17 controls the image capturing unit 14 such that reflected images of a ring pattern are captured at predetermined time intervals over a predetermined period (e.g., 15 seconds, etc.). As a result, the image capturing unit 14 repeatedly acquires the captured images of the reflected image. The number of times that an image is captured in one second may be, for example, five times, ten times, or the like. The acquired multiple captured images may be stored in an unshown storage medium.

(Step S104) The control unit 17 instructs the calculating unit 15 to calculate a bluntness level of each of the multiple captured images. According to the instruction, the calculating unit 15 calculates multiple bluntness levels for each captured image. When calculating the bluntness levels, the calculating unit 15 may or may not specify a center position of a reflected image of the ring pattern, for example, for each captured image. In the case of the latter, it is also possible that the center position specified in a first captured image is used as the center position in other captured images. The calculating unit 15 may calculate the total or a representative value of the multiple bluntness levels for each captured image.

(Step S105) The control unit 17 instructs the output unit 16 to perform output regarding the multiple bluntness levels calculated by the calculating unit 15. According to the instruction, the output unit 16 performs output regarding the multiple bluntness levels. The output unit 16 may output, for example, a graph showing a time-series change in the total of the bluntness levels calculated by the calculating unit 15. Then, the measurement regarding the lacrimal fluid layer on the cornea surface of the examination target eye 2 and the output of the measurement result are ended.

In the flowchart in FIG. 2, when an examinee closes his or her eyelid while an image of a reflected image of a ring pattern is being captured, the ring pattern cannot be detected, and thus an image captured when the eyelid is closed does not necessarily have to be used to calculate the bluntness level. The processing order in the flowchart in FIG. 2 is merely an example, and the order of the steps may be changed as long as similar effects can be obtained.

As described above, with the ophthalmic apparatus 1 according to this embodiment, it is possible to reduce the influence of a change between baselines of luminance values of an iris region and a pupil region, by calculating bluntness information using the shape of a peak of the luminance values of reflected images, and thus it is possible to realize proper measurement regarding the state of a lacrimal fluid layer on a cornea surface of the examination target eye 2. In particular, it is possible to reduce the influence according to a change in the pupil size when performing the measurement over a predetermined period, and thus it is possible to realize precise measurement. Furthermore, it is possible to acquire a time-series change in the bluntness information, by repeatedly capturing images of a ring pattern and calculating bluntness information of multiple captured images over time, and thus it is possible to see a time-series change regarding the state of a lacrimal fluid layer. If multiple pieces of bluntness information are calculated and output from one captured image, it is possible to see the state of a lacrimal fluid layer, at multiple points on a cornea surface of the examination target eye 2. If the state of a lacrimal fluid layer of an examination target eye is seen, for example, evaluation of dry eye syndrome and the like can be performed.

In this embodiment, a case was mainly described in which the ophthalmic apparatus 1 repeatedly captures images of the anterior eye part of the examination target eye 2 and outputs information indicating a time-series change in the bluntness information, but there is no limitation to this. For example, it is also possible that the ophthalmic apparatus 1 captures an image of the anterior eye part of the examination target eye 2 at a given point in time, and calculates bluntness information, using the captured image, at that image capturing point in time and outputs the bluntness information. At that time, it is also possible to calculate pieces of bluntness information at multiple positions in a captured image at a given point in time, or to calculate bluntness information at a measurement point. In the case of the latter, it is also possible to calculate bluntness information at a measurement point suited to observe the state of a lacrimal fluid layer of the examination target eye 2, for example.

Furthermore, in this embodiment, a case was mainly described in which the predetermined pattern that is projected onto the cornea surface of the examination target eye 2 is a pattern of multiple rings, but there is no limitation to this. For example, the predetermined pattern may also be a pattern having multiple lines arranged in a lattice, a pattern having multiple straight lines arranged in parallel, or the like, a pattern having multiple points arranged in a regular manner, or a pattern having one line, a pattern having one point, or the like. Also in such cases, it is possible to see the state of a lacrimal fluid layer, by calculating bluntness information of a maximum portion of luminance values, in any direction that is orthogonal to a line in the captured reflected image or direction that passes through the point.

Furthermore, in this embodiment, a case was described in which bluntness information regarding multiple points is calculated in one captured image, but, as can be seen from FIGS. 5(a) and 6, if the lacrimal fluid layer is broken, typically, a peak becomes blunt due to a ring being broken, but, in a portion in which a ring is incomplete, the peak may be kept sharp. Accordingly, it may be considered that data in a range in which the bluntness level is low does not properly indicate whether or not the lacrimal fluid layer is broken. On the other hand, it appears that data in a range in which the bluntness level is high properly indicates whether or not the lacrimal fluid layer is broken. Accordingly, in the case of outputting a representative value or the total regarding bluntness information, the total or the representative value of bluntness information in a range of predetermined values, for example, N pieces of bluntness information in descending order in bluntness level, may be output. Specifically, the total or the representative value of N bluntness levels in descending order or the total or the representative value of N sharpness levels in ascending order may be output. N is an integer of two or more. If the lacrimal fluid layer is broken in this manner, the dispersion level of the bluntness information at the positions of multiple peaks contained in one captured image increases. Accordingly, the output unit 16 may output a variance of multiple pieces of bluntness information, as output regarding the multiple pieces of bluntness information. In that case, it is indicated that the broken level of a lacrimal fluid layer is higher in accordance with an increase in the variance.

Furthermore, there may be an examinee whose eyelids gradually lower during the measurement. In such a case, the number of pieces of bluntness information that can be calculated in a measurement period gradually decreases. In such a situation, if information indicating a time-series change in the total of the bluntness information is output, the output is inadequate. For example, even when the bluntness information itself does not change over time, the total of bluntness levels decreases over time according to a decrease in the number of pieces of bluntness information. In order to avoid this problem, the calculating unit 15 may specify measurement points at which bluntness information can be acquired in all captured images captured from when the measurement is started to when the measurement is ended, and use only bluntness information at the specified measurement points, for example, to calculate the total of the bluntness information. For example, if the first to $M^{th}$ peaks (measurement points) from the center can be specified in all captured images on a straight line radially extending at an angle $\theta$, the first to $M^{th}$ pieces of bluntness information from the center may be calculated in that angular direction, in each captured image. This processing may be performed in a radial direction at each angle.

Furthermore, in the foregoing embodiment, each process or each function may be realized through centralized processing using a single apparatus or a single system, or may be realized through distributed processing using multiple apparatuses or multiple systems.

Furthermore, in the foregoing embodiment, information transmission performed between constituent elements may be such that, for example, if two constituent elements for transmitting information are physically different from each other, the transmission is performed by one of the constituent elements outputting the information and the other constituent element accepting the information, or alternatively, if two constituent elements for transmitting information are physically the same, the transmission is performed by shifting from a processing phase corresponding to one of the constituent elements to a processing phase corresponding to the other constituent element.

Furthermore, in the foregoing embodiment, information relating to the processing performed by each constituent element, for example, information that is to be accepted, acquired, selected, generated, transmitted, or received by each constituent element, information such as a threshold value, a numerical expression, or an address used by each constituent element in the processing and the like may be retained in an unshown storage medium temporarily or for a long period of time even if not specified in the description above. Furthermore, information may be accumulated in the unshown storage medium by each constituent element or by an unshown accumulating portion. Furthermore, information may be read from the unshown storage medium by each constituent element or by an unshown reading portion.

Furthermore, in the foregoing embodiment, if information used in each constituent element or the like, for example, information such as a threshold value, an address, or various setting values used by each constituent element in the processing may be changed by a user, the user may or may not be allowed to change such information as appropriate even though this is not specified in the description above. If the user is allowed to change such information, the change may be realized by, for example, an unshown accepting portion that accepts a change instruction made by the user and an unshown changing portion that changes information according to the change instruction. The change instruction may be accepted by the unshown accepting portion, for example, by accepting information from an input device, by receiving information transmitted via a communication line, or by accepting information read from a predetermined storage medium.

Furthermore, in the foregoing embodiment, each constituent element may be configured by dedicated hardware, or alternatively, constituent elements that can be realized by software may be realized by executing a program. For example, each constituent element may be realized by a program execution unit such as a CPU reading and executing a software program stored in a storage medium such as a hard disk or a semiconductor memory. When executing the program, the program execution unit may execute the program while accessing the storage unit or the storage medium. Furthermore, this program may be executed as a result of being downloaded from a server or the like, or may be executed by reading a program stored in a predetermined storage medium (e.g., an optical disk such as a CD-ROM, a magnetic disk, a semiconductor memory, etc.). Furthermore, the program may be used as a program forming a program product. Furthermore, a computer that executes the program may be a single computer or may be multiple computers. That is to say, centralized processing may be performed, or distributed processing may be performed.

The present invention is not limited to the embodiment set forth herein. Various modifications are possible within the scope of the invention.

Industrial Applicability

As described above, the ophthalmic apparatus according to the present invention has the effect of making it possible to reduce the influence of a change in luminance values in a pupil region, and thus this apparatus is useful as an ophthalmic apparatus for performing measurement regarding the state of a lacrimal fluid layer on a cornea surface of an examination target eye.

**Claims**

1. An ophthalmic apparatus for performing measurement regarding the state of a lacrimal fluid layer on a cornea surface of an examination target eye, comprising:

   a light-projecting unit that projects a predetermined pattern onto a cornea surface;
   an image capturing unit that captures a reflected image of the pattern reflected off the cornea surface;
   a calculating unit that calculates bluntness information indicating a bluntness level at a maximum portion of luminance values in the reflected image captured by the image capturing unit; and
   an output unit that performs output regarding the bluntness information calculated by the calculating unit.

2. The ophthalmic apparatus according to claim 1,
   wherein the image capturing unit repeatedly captures reflected images of the pattern,
   the calculating unit calculates bluntness information for each of the reflected images that were repeatedly captured, and
   the output unit outputs information indicating a time-series change in the bluntness information.

3. The ophthalmic apparatus according to claim 1 or 2,
   wherein the predetermined pattern is a ring pattern,
   the calculating unit calculates bluntness information at an intersection between the ring pattern and each straight line radially extending at every predetermined angle from a center position specified in a reflected image of the ring pattern, and
   the output unit performs output regarding the multiple pieces of bluntness information calculated by the calculating unit.

Examination target eye 2

Image capturing
unit 14

Light-projecting unit 13

Control unit 17

Calculating unit 15

Output unit 16

Ophthalmic apparatus 1

**FIG.1**

Start

S101
Perform alignment

S102
Alignment OK? — N

Y

S103
Capture image of pattern

S104
Calculate bluntness level

S105
Perform output
regarding bluntness level

End

**FIG.2**

**FIG.3**

(a)

(b)

(c)

FIG.4

(a)

(b)

(c)

**FIG.5**

Distance from center

**FIG.6**

FIG.7

Elapsed time

FIG.8

FIG.9

<div align="center">**INTERNATIONAL SEARCH REPORT**</div>

| International application No. |
|---|
| PCT/JP2019/025379 |

**A. CLASSIFICATION OF SUBJECT MATTER**
Int.Cl. A61B3/10(2006.01)i

According to International Patent Classification (IPC) or to both national classification and IPC

**B. FIELDS SEARCHED**

Minimum documentation searched (classification system followed by classification symbols)
Int.Cl. A61B3/00-3/12, 3/13-3/16

Documentation searched other than minimum documentation to the extent that such documents are included in the fields searched
Published examined utility model applications of Japan    1922-1996
Published unexamined utility model applications of Japan    1971-2019
Registered utility model specifications of Japan    1996-2019
Published registered utility model applications of Japan    1994-2019

Electronic data base consulted during the international search (name of data base and, where practicable, search terms used)
JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | JP 2006-204773 A (TOMEY CORPORATION) 10 August 2006, paragraphs [0006], [0019]-[0029] & US 2008/0309872 A1, paragraphs [0009], [0054]-[0066] & WO 2006/080217 A1 & EP 1844702 A1 | 1-3 |
| Y | JP 2005-230328 A (TOPCON CORPORATION) 02 September 2005, paragraphs [0003], [0029]-[0032] (Family: none) | 1-3 |

☒ Further documents are listed in the continuation of Box C.    ☐ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 03.09.2019 | 17.09.2019 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2019/025379 |

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2003-47593 A (CANON INC.) 18 February 2003, paragraphs [0024]-[0027] (Family: none) | 1-3 |
| A | JP 2000-254099 A (CANON INC.) 19 September 2000, paragraphs [0017]-[0019] (Family: none) | 1-3 |
| A | JP 2006-167002 A (TOMEY CORPORATION) 29 June 2006, paragraphs [0004], [0005], [0018] (Family: none) | 1-3 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006204773 A